# EUROPEAN PATENT SPECIFICATION

(11) **EP 3 916 387 B1**
(45) Date of publication and mention of the grant of the patent: **08.05.2024**
(21) Application number: 20919367.1
(22) Date of filing: 07.09.2020
(51) Int. Cl.: G01N 33/543

(54) **LATERAL FLOW ASSAY DEVICE FOR TRAUMATIC BRAIN INJURY DIAGNOSIS USING TIME-RESOLVED FLUORESCENCE ANALYSIS, AND METHOD FOR DIAGNOSING TRAUMATIC BRAIN INJURY BY USING SAME**
LATERAL-FLOW-ASSAYVORRICHTUNG FÜR DIE DIAGNOSE VON HIRNTRAUMATA MITTELS EINER ZEITAUFGELÖSTEN FLUORESZENZANALYSE UND VERFAHREN ZUR DIAGNOSE VON HIRNTRAUMATA UNTER VERWENDUNG DERSELBEN
DISPOSITIF DE DOSAGE À ÉCOULEMENT LATÉRAL POUR DIAGNOSTIC DE LÉSION CÉRÉBRALE TRAUMATIQUE À L'AIDE D'UNE ANALYSE DE FLUORESCENCE À RÉSOLUTION TEMPORELLE, ET PROCÉDÉ DE DIAGNOSTIC D'UNE LÉSION CÉRÉBRALE TRAUMATIQUE AU MOYEN DE CETTE DERNIÈRE

(30) Priority: 01.04.2020 KR 20200039763
(43) Date of publication of application: 01.12.2021
(73) Proprietor: Precision Biosensor Inc., Daejeon 34036 (KR)
(72) Inventor: KANG, Jongeun, Chungcheongbuk-do 28113 (KR); HWANG, Kyu-Youn, Sejong-si 30064 (KR); PARK, Jong-Myeon, Seongnam-si, Gyeonggi-do 13589 (KR); KIM, Hanshin, Anyang-si, Gyeonggi-do 14106 (KR)
(74) Representative: Gevers Patents
(86) International application number: PCT/KR2020/012035
(87) International publication number: WO 2021/201350

(56) References cited:
- WO-A2-2010/148391
- JP-A- 2016 533 499
- JP-A- 2017 509 902
- KR-A- 20050 062 531
- KR-A- 20120 132 249
- KR-A- 20180 025 295
- KR-A- 20190 117 350
- VENKATNARAYANAN R ET AL: "a highly sensitive point of care test for GFAP-a brain biomarker in serum", CLINICAL CHEMISTRY, vol. 63, no. Supplement 1, 3 August 2017 (2017-08-03), pages S272-S273, XP055970028, US ISSN: 1530-8561
- GAO XUEFEI ET AL: "Paper-Based Surface-Enhanced Raman Scattering Lateral Flow Strip for Detection of Neuron-Specific Enolase in Blood Plasma", ANALYTICAL CHEMISTRY, vol. 89, no. 18, 1 September 2017 (2017-09-01), pages 10104-10110, XP055970143, US ISSN: 0003-2700, DOI: 10.1021/acs.analchem.7b03015 Retrieved from the Internet: URL:https://pubs.acs.org/doi/pdf/10.1021/a cs.analchem.7b03015>

## Description

### [Technical Field]

The present invention relates to a lateral flow assay device for diagnosing traumatic brain injury using time-resolved fluorescence analysis and a method for diagnosing traumatic brain injury using the same. More particularly, the present invention relates to a lateral flow assay device for diagnosing traumatic brain injury using time-resolved fluorescence analysis and a method for diagnosing traumatic brain injury using the same capable of providing high sensitivity even if the blood concentration of a glial fibrillary acidic protein as a mild traumatic brain injury (mTBI) marker is low.

### [Background Art]

Brain injury is proud of a globally high incidence, and has problems in that a large number of CT scans for brain injury, particularly mild traumatic brain injury (mTBI) is not helped to determine meaningful brain injury after performing a general computer tomography (CT) scan, radiation exposure is not only large, but also space, time, and costly limitations are large.

Recently, some studies have been reported by targeting an increase in need of field diagnosis capable of reducing unnecessary CT scan number and more rapidly leaving the hospital by screening all patients with mild traumatic brain injury (mTBI) through a simple blood test (Berger et al., 2007; Poli-de-Figueiredo et al., 2006).

However, until now, there is reported no high-reliable bio-assay that may appropriately classify patients.

In order to determine the severity of the brain injury, a bio-assay capable of accurately measuring a trace amount of biomarker in a blood sample with high sensitivity is required, and it should also be applicable to field diagnosis such as emergency rooms.

Related technologies are disclosed in: Venkatnarayanan R., et al.: "a highly sensitive point of care test for GFAP-a brain biomarker in serum", CLINICAL CHEMISTRY, vol. 63, no. Supplement 1, 3 August 2017, pages S272-S273, ISSN: 1530-8561; Gao Xuefei, et al.: "Paper-Based Surface-Enhanced Raman Scattering Lateral Flow Strip for Detection of Neuron-specific Enolase in Blood Plasma", ANALYTICAL CHEMISTRY, vol. 89, no. 18, 1 September 2017, pages 10104-10110, ISSN: 0003*2700, DOI: 10.1021/acs.analchem.7b03015; and WO 2010/148391 A2.

### [Disclosure]

### [Technical Problem]

The present invention has been made in an effort to provide a lateral flow assay device for diagnosing traumatic brain injury using time-resolved fluorescence analysis and a method for diagnosing traumatic brain injury using the same capable of measuring the blood concentration of a glial fibrillary acidic protein (GFAP) using a lateral flow immune assay device with high sensitivity to detect or/and classify any brain-related traumatic severity and being useful for diagnosis and having high reliability.

### [Technical Solution]

Exemplary embodiments of the present invention are set out in the appended claims.

### [Advantageous Effects]

According to an exemplary embodiment of the present invention, it is possible to measure at least GFAP concentration in the blood using a lateral flow immune assay device with high sensitivity to detect or/and classify any brain-related traumatic severity, particularly, mild traumatic brain injury and to provide a lateral flow assay device for field diagnosis of traumatic brain injury and a manufacturing method thereof useful for diagnosis of traumatic brain injury and with high reliability.

### [Description of the Drawings]

FIG. 1 is a diagram illustrating a schematic diagram and an analysis principle of a lateral flow immune assay device for detecting a traumatic brain injury biomarker according to an exemplary embodiment of the present invention.
FIG. 2 is a flowchart for describing a method for diagnosing traumatic brain injury using a lateral flow assay device for field diagnosis of traumatic brain injury according to an exemplary embodiment of the present invention.
FIG. 3 is a graph of confirming non-specific reaction reduction in the case of using a mouse-origin antibody and in the case of using mouse and rabbit-origin antibodies together in various GFAP negative plasma samples.
FIG. 4 is a lateral flow assay sensor photograph showing a non-specific phenomenon according to a combination of mouse and rabbit-origin antibody pairs when introducing GFAP negative plasma.
FIG. 5 is a graph showing a difference in fluorescence signal for each GFAP concentration according to a combination of mouse and rabbit-origin antibody pairs.
FIG. 6 is a graph showing a difference in fluorescence signal for each GFAP concentration in plasma.
FIG. 7 is a graph showing an ELISA test result for each GFAP concentration in plasma.

### [Mode for Invention]

Hereinafter, a lateral flow assay device for field diagnosis of traumatic brain injury according to an exemplary embodiment of the present invention and a manufacturing method thereof will be described with reference to the accompanying drawings.

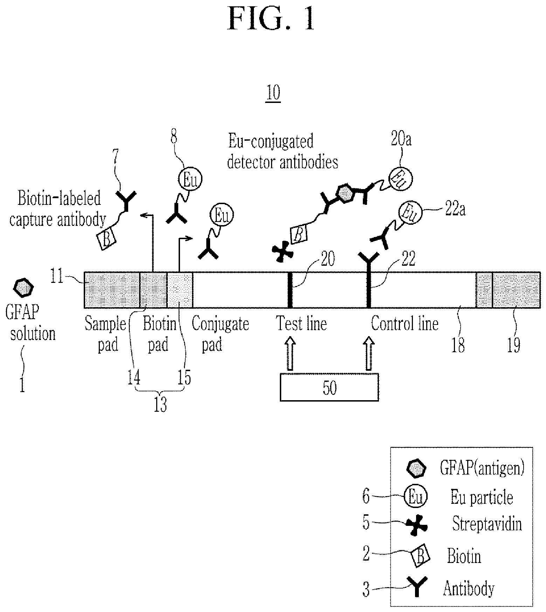

Referring to FIG. 1, a lateral flow assay device 10 for field diagnosis of traumatic brain injury according to an exemplary embodiment of the present invention includes a sample pad 11 into which a test sample containing an analyte (including a biomarker for diagnosing traumatic brain injury) is injected, an adsorption pad 13 including a probe which is mixed to the analyte moving from the sample pad 11 to form an analyte complex, a porous film 18 which fluid-communicates with the adsorption pad 13 and capillary-migrates the analyte complex from the adsorption pad 13 to a detection line 20, and an absorption pad 19 which is formed at an end of the porous film 18 to promote capillary action and fluid flow and accommodates waste after analysis, in which the sample pad 11, the adsorption pad 13, the porous film 18, and the absorption pad 19 may be supported by a rigid support.

Here, the "probe" includes a capture antibody 7 consisting of an antibody 3 specifically binding to any one of traumatic brain injury markers included in the analyte. According to the invention, the traumatic brain injury marker is glial fibrillary acidic protein (GFAP) (the probe is confused to mean a marker) and any one of: S100B, UCH-L1, NSE, NeuN, CNPase, CAM-1, iNOS, MAP-1, MAP-2, SBDP145, SBDP120, III-tubulin, synaptic protein, neuroserpin, internexin, LC3, neurofacin, EAAT, DAT, nestin, cortin-1, CRMP, ICAM-1, ICAM-2, ICAM-5, VCAM-1, NCAM-1, NCAM-L1, NCAM-120, NCAM-140, NL-CAM, AL-CAM, or C-CAM1, and a detector antibody 8 consisting of a fluorescent material 6 binding to the antibody 3.

In the lateral flow assay device 10 for field diagnosis of traumatic brain injury according to the exemplary embodiment of the present invention, the adsorption pad 13 may have first and second adsorption pads 14 and 15 sequentially provided by the probe. The first adsorption pad 14 may provide a specific binding material 2 forming the capture antibody 7 binding to the antibody 3 forming the analyte complex and the second adsorption pad 15 may provide the fluorescent material 6 forming the detector antibody 8 to provide a fluorescent marker to the analyte complex.

A capture material 5 capable of selectively binding to the binding material 2 included in the capture antibody 7 is immobilized on the detection line 20.

While the traumatic brain injury marker passes through the sample pad 11 and the adsorption pad 13, the capture antibody 7 labeled with the specific binding material 2 binds to the detector antibody 8 labeled with the fluorescent material 6 to form a specific analyte complex 20a and the specific analyte complex 20a is immobilized on the detection line 20 coated with the capture material 5 by interaction between the binding material 2 and the capture material 5.

In the lateral flow assay device 10 for field diagnosis of traumatic brain injury according to the exemplary embodiment of the present invention, the detection line 20 may include an antigen, a hapten, an antibody, a protein A, or G, avidin, streptavidin, a secondary antibody, and a biological capture material including a complex thereof.

In this specification, the biological capture material used streptavidin, and it is preferred to specifically bind to biotin which is the specific binding material 2 of the probe.

The capture material serves to provide a fixed binding site to the specific analyte complex 20a. In some examples, the analyte such as an antibody, an antigen and the like have two binding sites.

The detection line 20 is disposed in a line form in a direction substantially perpendicular to the flow of the sample, and the detection line 20 may indicate the presence of the analyte, but it is difficult to often measure the concentration of the analyte in the test sample by using only the detection line 20. Therefore, on the porous film 18, a control line 22 located on the downstream of the detection line 20 is provided.

The control line 22 may be provided with a capture material that may bind to any probe passing through the porous film 18.

In particular, any probes 22a that do not bind to the analyte are bound and fixed with the capture material of the control line 22 through the detection line 20.

The capture material used in the control line 22 may be different from the capture material 5 used in the detection line 20.

A probe fluorescent signal in the detection line 20 and the control line 22 may be measured using a time-resolved fluorescence tester 50.

The time-resolved fluorescence tester 50 is configured to simultaneously irradiate pulse excitation light to the detection line 20 and the control line 22, and may receive fluorescent signals emitted from the fluorescent material of the detection line 20 and the control line 22 at the same time.

The time-resolved fluorescence tester 50 may use one or more pulsed excitation sources and photodetectors that are linked with any other components such as an optical filter.

According to the invention, the fluorescent material has a long emission lifetime of 1 microsecond or more. Europium (Eu(III)) is used as the fluorescent material and has both a relatively long emission lifetime and a large stoke migration so as to substantially remove background interference such as scattering light and self-fluorescence.

Therefore, the time-resolved fluorescence tester 50 may have a simple and inexpensive design. For example, the time-resolved fluorescence tester 50 may excite the fluorescent material using a light emitting diode (LED) and may also detect the fluorescence of the detection line 20 and the control line 22 without using an expensive component such as a monochrometer or a narrow emission bandwidth optical filter.

Meanwhile, since the glial fibrillary acidic protein (GAFP) which is one of markers for field diagnosis of mild traumatic brain injury (mTBI) has a low blood concentration, in order to increase the sensitivity of the lateral flow assay device 10 for diagnosing traumatic brain injury according to the exemplary embodiment of the present invention, the time-resolved fluorescence analysis was performed by varying a capture/detector antibody pair binding to the GAFP.

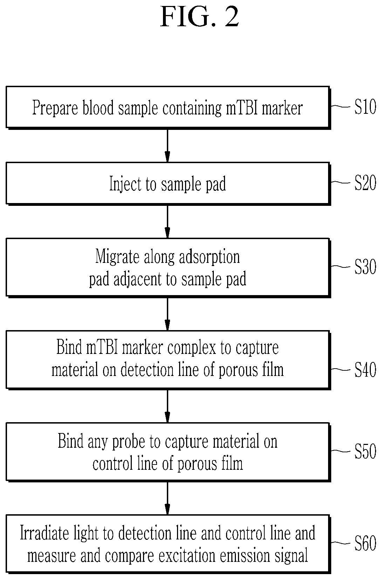

FIG. 2 is a flowchart for describing a method for diagnosing traumatic brain injury using a lateral flow assay device for diagnosing traumatic brain injury according to an exemplary embodiment of the present invention.

### [Experimental Example 1]

A method for diagnosing traumatic brain injury using a lateral flow assay device for diagnosing traumatic brain injury according to an exemplary embodiment of the present invention is to detect a biomarker, GFAP in a sample using a lateral flow analysis method and a time-resolved fluorescence technique, and includes the steps of preparing a blood sample containing a mild traumatic brain injury (mTBI) marker (S10), injecting the blood sample containing the mild traumatic brain injury marker into a sample pad 11 (S20), forming a traumatic brain injury marker complex 20a consisting of a capture antibody 7 labeled with a specific binding material 2 and a detector antibody 8 labeled with a fluorescent material 6 while migrating the blood sample containing the traumatic brain injury marker along an adsorption pad 13 adjacent to the sample pad 11 with a capillary phenomenon (S30), migrating the traumatic brain injury marker complex 20a along a porous film 18 fluid-communicating with the adsorption pad 13 to bind to a capture material 5 on a detection line 20 of the porous film 18 (S40), binding any probe 22b not binding to the traumatic brain injury marker to a capture material of a control line 22 through the detection line 20 (S50), and measuring a concentration of the traumatic brain injury marker by irradiating light to the detection line 20 and the control line 22 from a time-resolved fluorescence tester 50 and comparing fluorescent signals of the detection line 20 and the control line 22 to diagnose the traumatic brain injury (S60).

A time-resolved fluorescence immune analysis method for detecting the presence or amount of GFAP in a test sample may include the steps of:
i) disposing a time-resolved fluorescence tester 50 by approaching a detection line 20, in which the time-resolved fluorescence tester 50 includes a pulse excitation source and a time-gated detector;
ii) emitting a detection signal from the fluorescent material binding to the GFAP by exciting the fluorescent material in the detection line 20 as the pulse excitation source; and
iii) analyzing the intensity of the detection signal with a time-gated detector.

First, various GFAP-negative plasma samples (GFAP level to 0 pg/mL) are injected to the lateral flow assay device 10 for field diagnosis of traumatic brain injury according to an exemplary embodiment of the present invention and then the fluorescence intensity was measured by using a time-resolved fluorescence measuring method.

As a result of measuring the corresponding samples through an ELISA kit (Creative Diagnostics, USA) sold on the market, it was confirmed that a very small optical signal OD at a buffer level was measured in all the samples.

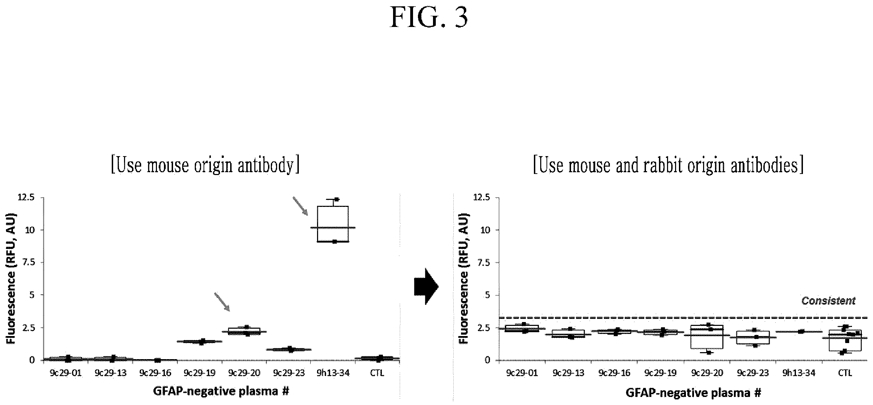

FIG. 3 is a graph of confirming non-specific reaction reduction in the case of using a mouse-origin antibody and in the case of using mouse and rabbit-origin antibodies at the same time in various GFAP negative plasma samples.

As shown in FIG. 3, in the case of using a mouse-origin antibody, non-specific characteristics are exhibited in a specific GFAP negative plasma sample, but in the case of using mouse and rabbit-origin antibodies at the same time, it may be seen that constant fluorescence intensities are shown in all the GFAP negative plasma samples and the non-specific reaction is reduced.

Accordingly, in accordance with the invention, when in the adsorption pad 13, the specific binding material 2 uses biotin, the fluorescent material uses europium (Eu), and the antibody 3 forming the capture body 7 by binding to the biotin and the antibody 3 forming the detector antibody 8 by binding to the fluorescent material 6 use a mouse-origin antibody and a rabbit-origin antibody at the same time, it may be seen that it is preferred to exhibit the constant intensity and reduce the non-specific reaction.

This is because when only the rabbit-origin antibody is applied, it is difficult to implement the sensitivity, but when the rabbit-origin antibody and the mouse-origin antibody are simultaneously used, the sensitivity of a low concentration section may be secured.

The traumatic brain injury biomarker uses GFAP and any one of: S100B, UCH-L1, NSE, NeuN, CNPase, CAM-1, iNOS, MAP-1, MAP-2, SBDP145, SBDP120, III-tubulin, synaptic protein, neuroserpin, α-internexin, LC3, neurofacin, EAAT, DAT, nestin, corin-1, CRMP, ICAM-1, ICAM-2, ICAM-5, VCAM-1, NCAM-1, NCAM-L1, NCAM-120, NCAM-140, NL-CAM, AL-CAM, or C-CAM1.

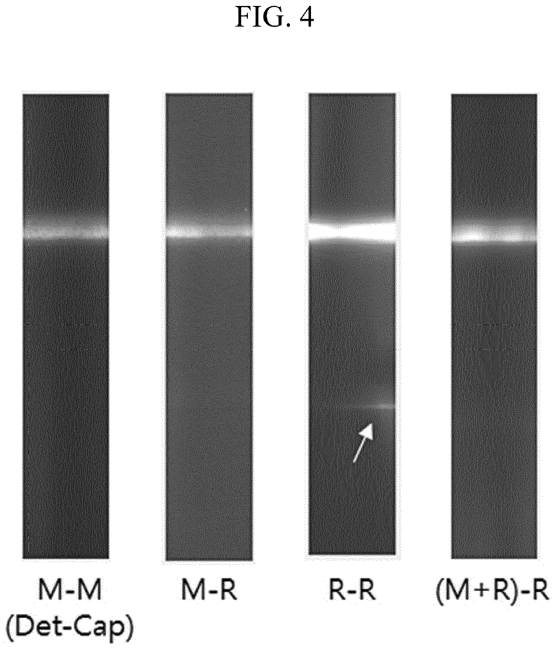

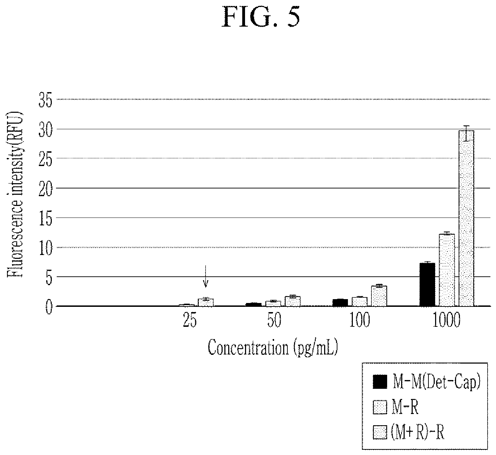

Then, the non-specific phenomenon according to an antibody origin was experimented with reference to FIGS. 4 and 5.

### [Experimental Example 2]

A lateral flow sensor according to an antibody origin was manufactured to experiment a non-specific phenomenon and sensitivity according to the antibody origin. To prepare plasma samples with various GFAP concentrations, a GFAP material was purchased from Hytest Co., Ltd. and diluted continuously in GFAP negative plasma. It was confirmed that the concentration of the manufactured GFAP sample was accurate by using a commercial ELISA kit (Creative Diagnostics, USA).

FIG. 4 is a lateral flow assay sensor photograph showing a non-specific phenomenon according to a combination of mouse and rabbit-origin antibody pairs when introducing GFAP negative plasma and FIG. 5 is a graph showing a difference in fluorescence signal for each GFAP concentration according to a combination of mouse and rabbit-origin antibody pairs.

Here, a pair of the detector antibody 8 and the capture antibody 7 is represented by (Det-Cap), a mouse-origin antibody is represented by M, a rabbit-origin antibody is represented by R, and a mixture of mouse and rabbit-origin antibodies is represented by M+R.

Referring to FIG. 4, with respect of the pair (Det-Cap) of the detector antibody 8 and the capture antibody 7, in the case (R-R) of using only a rabbit-origin antibody, it may be confirmed that the non-specific signal is severely shown in a band form for the GFAP negative plasma.

Referring to FIG. 5, it may be seen that in a combination ((M+R)-R) of using a mixture of a mouse-origin antibody and a rabbit-origin antibody as the detector antibody 8 and using the rabbit-origin antibody as the capture antibody 7, a difference in fluorescence signal size according to a GFAP concentration and a fluorescence signal size even at a low concentration (25 pg/mL) are largest than a case (M-M) of using only the mouse-origin antibody as a pair of the detector antibody 8 and the capture antibody 7 or a case (M-R) of using the mouse-origin antibody and the rabbit-origin antibody as a pair of the detector antibody 8 and the capture antibody 7, respectively.

When comparing the case (M-M) of using only the mouse-origin antibody as the pair of the detector antibody 8 and the capture antibody 7 or the case (M-R) of using the mouse-origin antibody and the rabbit-origin antibody as the detector antibody 8 and the capture antibody 7, respectively, it may be seen that in the case (M-R) of using the mouse-origin antibody and the rabbit-origin antibody as the pair of the detector antibody 8 and the capture antibody 7, respectively, the fluorescence signal is excellent.

In the lateral flow assay device 10 for field diagnosis of traumatic brain injury according to the exemplary embodiment of the present invention, as illustrated in FIGS. 4 and 5, in the case (M-M) of using the mouse-origin antibody as the detector antibody 8 and the capture antibody 7, respectively, it may be seen that the sensitivity is lowered and there is a limitation in measurement of GFAP concentration to determine the brain injury severity.

Further, in the case of using a rabbit-origin antibody which is generally known that antibody-antigen reactivity is 10 to 100 times higher than the mouse-origin antibody, as the detector antibody 8 and the capture antibody 7, respectively, it may be seen that since non-specific bands are generated on a lateral flow sensor to distort a signal, it is not preferred to measure the concentration of the GFAP which is the traumatic brain injury biomarker.

Further, when the mouse-origin antibody is used as the detector antibody 8 and the rabbit-origin antibody is used as the capture antibody 7, it may be seen that non-specific bands are not generated and the sensitivity is partially increased as compared with when using the mouse-origin antibody alone, but it is not enough to determine a concentration level that may determine brain injury severity using GFAP.

Accordingly, in the case (M+R)-R) of using the mixture of the mouse-origin antibody and the rabbit-origin antibody as the detector antibody 8 and using the rabbit-origin antibody as the capture antibody 7, it may be seen that since the sufficient sensitivity may be implemented, it is preferred to measure the concentration of the traumatic brain injury biomarker.

### [Experimental Example 3]

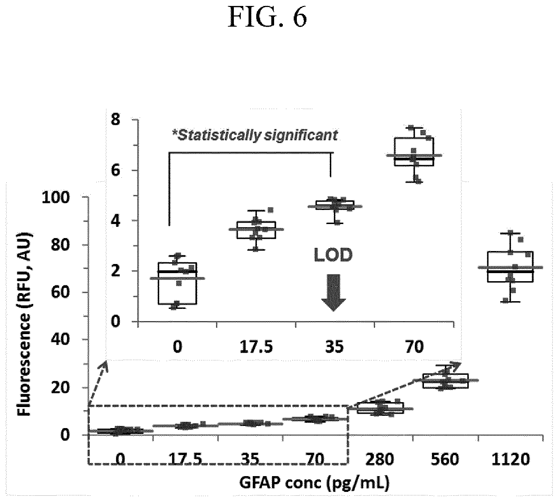

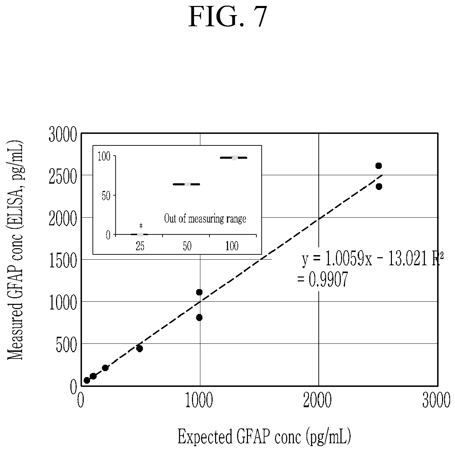

Hereinafter, GFAP measuring sensitivity using the lateral flow assay device for field diagnosis of traumatic brain injury according to the exemplary embodiment of the present invention will be described with reference to FIGS. 6 and 7.

FIG. 6 is a graph showing a result of measuring various GFAP concentrations using a lateral flow assay device for field diagnosis of traumatic brain injury according to an exemplary embodiment of the present invention and FIG. 7 is a graph showing a result of confirming the GFAP concentrations of FIG. 6 using an ELISA method.

In the lateral flow assay device for field diagnosis of traumatic brain injury according to the exemplary embodiment of the present invention, it may be seen that when in the content of a rabbit-origin antibody binding with europium nanoparticles, the detector antibody concentration is lowered to 0.1% to less than 2%, preferably 1% in an adsorption pad spray solution so that the non-specific bands are not generated, the sensitivity of a low concentration section between 20 pg/mL to 30 pg/mL is secured, but a signal deviation according to a concentration occurs.

As a result, when a mouse-origin antibody binding with europium nanoparticles is added to the adsorption pad spray solution at a concentration 3% to 12%, preferably 3% and the rabbit and mouse-origin antibodies are applied at the same time, the signal is secured even in the GFAP low-concentration section and the signal deviation according to the concentration is also improved.

As illustrated in FIG. 6, even in the GFAP concentration capable of determining the brain injury severity of less than about 100 pg/mL, preferably less than about 50 pg/mL, the sensitivity is provided to provide a lateral flow assay device for field diagnosis of traumatic brain injury with TRF-based high sensitivity.

In FIG. 7, it was confirmed through a commercial ELISA kit that the GFAP sample concentration used in the experiment was accurate. It was confirmed that the deviation between a manufacturing estimated concentration and an ELISA measurement concentration value was low, and the correlation was 0.99 or more.

Since the lateral flow assay device 10 for field diagnosis of traumatic brain injury according to the exemplary embodiment of the present invention exhibits the sensitivity even at a low-concentration biomarker GAFP concentration of 100 pg/mL, preferably 50 pg/mL for diagnosis of traumatic brain injury as described above, it may be seen that it is effective to diagnose the traumatic brain injury in combination with a Glasgow coma scale (GCS) (awake, language function, and exercise function).

### [Industrial Applicability]

According to an exemplary embodiment of the present invention, it is possible to measure at least GFAP concentration in the blood using a lateral flow immune assay device with high sensitivity to detect or/and classify any brain-related traumatic severity, particularly, mild traumatic brain injury and to provide a lateral flow assay device for field diagnosis of traumatic brain injury and a manufacturing method thereof useful for diagnosis of traumatic brain injury and with high reliability.

## Claims

1. A time-resolved fluorescence lateral flow assay device (10) comprising:
in a lateral flow assay device capable of detecting a traumatic brain injury marker,
a sample pad (11) into which a blood sample containing a traumatic brain injury marker is injected,
an adsorption pad (13) including a probe which is mixed to the marker when the traumatic brain injury marker moves from the sample pad to form a traumatic brain injury marker complex, and
a porous film (18) which fluid-communicates with the adsorption pad and capillary-migrates the traumatic brain injury marker complex from the adsorption pad to a detection line (20),
wherein the probe includes a capture antibody (7) consisting of an antibody (3) labeled with a specific binding material specifically binding to the traumatic brain injury marker and a detector antibody (8) consisting of an antibody labeled with a fluorescent material (6) having a relatively long emission lifetime of 1 microsecond or more,
wherein the traumatic brain injury marker is a glial fibrillary acidic protein (GFAP), and any one of S100B, UCH-L1, NSE, NeuN, CNPase, CAM-1, iNOS, MAP-1, MAP-2, SBDP145, SBDP120, III-tubulin, synaptic protein, neuroserpin, internexin, LC3, neurofacin, EAAT, DAT, nestin, cortin-1, CRMP, ICAM-1, ICAM-2, ICAM-5, VCAM-1, NCAM-1, NCAM-L1, NCAM-120, NCAM-140, NL-CAM, AL-CAM, or C-CAM1,
wherein biotin is used as the specific binding material, europium is used as the fluorescent material, and a mixture of a mouse-origin antibody and a rabbit-origin antibody is used as an antibody for the detector antibody.

2. The lateral flow assay device for traumatic brain injury using time-resolved fluorescence analysis of claim 1, wherein
the adsorption pad includes first (14) and second (15) adsorption pads sequentially provided by the probe, wherein the first adsorption pad provides a specific binding material (2) forming the capture antibody and the second adsorption pad provides the fluorescent material forming the detector antibody.

3. The lateral flow assay device for traumatic brain injury using time-resolved fluorescence analysis of claim 1, wherein
a mixture of at least two different kinds-origin antibodies is used as the antibody labeled with the specific binding material or the fluorescent material.

4. The lateral flow assay device for traumatic brain injury using time-resolved fluorescence analysis of claim 1, wherein
in an antibody bound with europium particles, the content of the rabbit-origin antibody is at a concentration of 0.1% to less than 2%, preferably 1% in an adsorption pad spray solution.

5. The lateral flow assay device for traumatic brain injury using time-resolved fluorescence analysis of claim 4, wherein
in the antibody bound with europium particles, the mouse-origin antibody is further added at a concentration of 3% to 12%, preferably 3% in the adsorption pad spray solution.

6. The lateral flow assay device for traumatic brain injury using time-resolved fluorescence analysis of claim 5, wherein
GFAP which is one of traumatic brain injury markers is detected with sensitivity of less than 100 pg/mL using the rabbit-origin antibody as the capture antibody.

7. A method for diagnosing traumatic brain injury using a lateral flow assay device (10) using time-resolved fluorescence analysis according to any one of claims 1 to 6, the method comprising the steps of:
preparing (S10) a blood sample containing a traumatic brain injury marker,
injecting (S20) the blood sample containing the traumatic brain injury marker into a sample pad (11),
forming (S30) a traumatic brain injury marker complex (20a) consisting of a capture antibody (7) labeled with a specific binding material (2) and a detector antibody (8) labeled with a fluorescent material (6) while migrating the blood sample containing the traumatic brain injury marker along an adsorption pad (13) adjacent to the sample pad with a capillary phenomenon,
migrating (S40) the traumatic brain injury marker complex along a porous film (18) fluid-communicating with the adsorption pad to bind to a capture material (5) on a detection line (20) of the porous film,
binding (S50) any probe (22b) not binding to the traumatic brain injury marker to a capture material of a control line (22) through the detection line, and
measuring (S60) a concentration of the traumatic brain injury marker by irradiating light to the detection line and the control line from a time-resolved fluorescence tester (50) and comparing fluorescent signals of the detection line and the control line to diagnose the traumatic brain injury.

8. The method of claim 7, comprising:
diagnosing the traumatic brain injury in combination with a Glasgow coma scale (GCS) (awake, language function, and exercise function).

## Patentansprüche

1. Eine Zeitaufgelöste Fluoreszenz-Lateral-Flow-Assayvorrichtung (10), umfassend:
in einer Lateral-Flow-Assayvorrichtung, die in der Lage ist, einen Schädel-Hirn-Trauma-Marker zu detektieren,
ein Probenkissen (11), in das eine Blutprobe, die einen Schädel-Hirn-Trauma-Marker enthält, injiziert wird,
ein Adsorptionskissen (13), das eine Sonde beinhaltet, die mit dem Marker gemischt wird, wenn sich der Schädel-Hirn-Trauma-Marker vom Probenkissen bewegt, um einen Schädel-Hirn-Trauma-Markerkomplex zu bilden, und
einen porösen Film (18), der strömungstechnisch mit dem Adsorptionskissen kommuniziert und den Schädel-Hirn-Trauma-Markerkomplex kapillar vom Adsorptionskissen zu einer Detektionslinie (20) migriert,
wobei die Sonde einen Fänger-Antikörper (7), bestehend aus einem Antikörper (3), der mit einem spezifischen Bindungsmaterial markiert ist, das spezifisch an den Schädel-Hirn-Trauma-Marker bindet, und einen Detektor-Antikörper (8), bestehend aus einem Antikörper, der mit einem fluoreszierenden Material (6) markiert ist, das eine relativ lange Emissionslebensdauer von 1 Mikrosekunde oder mehr aufweist, beinhaltet,
wobei der Schädel-Hirn-Trauma-Marker ein saures Gliafaserprotein (GFAP) und eines von S100B, UCH-L1, NSE, NeuN, CNPase, CAM-1, iNOS, MAP-1, MAP-2, SBDP145, SBDP120, III-Tubulin, synaptischem Protein, Neuroserpin, Internexin, LC3, Neurofacin, EAAT, DAT, Nestin, Cortin-1, CRMP, ICAM-1, ICAM-2, ICAM-5, VCAM-1, NCAM-1, NCAM-L1, NCAM-120, NCAM-140, NL-CAM, AL-CAM oder C-CAM1 ist,
wobei Biotin als spezifisches Bindungsmaterial verwendet wird, Europium als fluoreszierendes Material verwendet wird, und ein Gemisch aus einem Maus-Antikörper und einem Kaninchen-Antikörper als Antikörper für den Detektor-Antikörper verwendet wird.

2. Die Lateral-Flow-Assayvorrichtung für Schädel-Hirn-Traumata unter Verwendung von zeitaufgelöster Fluoreszenzanalyse nach Anspruch 1, wobei
das Adsorptionskissen ein erstes (14) und ein zweites (15) Adsorptionskissen beinhaltet, die nacheinander von der Sonde bereitgestellt werden, wobei das erste Adsorptionskissen ein spezifisches Bindungsmaterial (2) bereitstellt, das den Fänger-Antikörper bildet, und das zweite Adsorptionskissen das fluoreszierende Material bereitstellt, das den Detektor-Antikörper bildet.

3. Die Lateral-Flow-Assayvorrichtung für Schädel-Hirn-Traumata unter Verwendung von zeitaufgelöster Fluoreszenzanalyse nach Anspruch 1, wobei
ein Gemisch aus mindestens zwei Antikörpern unterschiedlicher Herkunft als mit dem spezifischen Bindungsmaterial oder dem fluoreszierenden Material markierter Antikörper verwendet wird.

4. Die Lateral-Flow-Assayvorrichtung für Schädel-Hirn-Traumata unter Verwendung von zeitaufgelöster Fluoreszenzanalyse nach Anspruch 1, wobei
bei einem mit Europiumpartikeln gebundenen Antikörper der Gehalt des Kaninchen-Antikörpers bei einer Konzentration von 0,1 % bis weniger als 2 %, bevorzugt 1 %, in einer Adsorptionskissen-Sprühlösung liegt.

5. Die Lateral-Flow-Assayvorrichtung für Schädel-Hirn-Traumata unter Verwendung von zeitaufgelöster Fluoreszenzanalyse nach Anspruch 4, wobei
bei dem mit Europiumpartikeln gebundenen Antikörper der Maus-Antikörper weiter mit einer Konzentration von 3 % bis 12 %, bevorzugt 3 %, in der Adsorptionskissen-Sprühlösung zugegeben wird.

6. Die Lateral-Flow-Assayvorrichtung für Schädel-Hirn-Traumata unter Verwendung von zeitaufgelöster Fluoreszenzanalyse nach Anspruch 5, wobei
GFAP, der einer der Schädel-Hirn-Trauma-Marker ist, mit einer Empfindlichkeit von weniger als 100 pg/mL unter Verwendung des Kaninchen-Antikörpers als Fänger-Antikörper detektiert wird.

7. Ein Verfahren zum Diagnostizieren von Schädel-Hirn-Traumata unter Verwendung einer Lateral-Flow-Assayvorrichtung (10) unter Verwendung von zeitaufgelöster Fluoreszenzanalyse nach einem der Ansprüche 1 bis 6, wobei das Verfahren die folgenden Schritte umfasst:
Vorbereiten (S10) einer Blutprobe, die einen Schädel-Hirn-Trauma-Marker enthält,
Injizieren (S20) der Blutprobe, die den Schädel-Hirn-Trauma-Marker enthält, in ein Probenkissen (11),
Bilden (S30) eines Schädel-Hirn-Trauma-Markerkomplexes (20a), bestehend aus einem mit einem spezifischen Bindungsmaterial (2) markierten Fänger-Antikörper (7) und einem mit einem fluoreszierenden Material (6) markierten Detektor-Antikörper (8), während des Migrierens der den Schädel-Hirn-Trauma-Marker enthaltenden Blutprobe entlang eines Adsorptionskissens (13), das an das Probenkissen angrenzt, mit einem Kapillarphänomen,
Migrieren (S40) des Schädel-Hirn-Trauma-Markerkomplexes entlang eines porösen Films (18), der strömungstechnisch mit dem Adsorptionskissen kommuniziert, um an ein Fängermaterial (5) an einer Detektionslinie (20) des porösen Films zu binden,
Binden (S50) jeder Sonde (22b), die nicht an den Schädel-Hirn-Trauma-Marker bindet, an ein Fängermaterial einer Kontrolllinie (22) durch die Detektionslinie, und
Messen (S60) einer Konzentration des Schädel-Hirn-Trauma-Markers durch Strahlen von Licht von einem zeitaufgelösten Fluoreszenztester (50) auf die Detektionslinie und die Kontrolllinie und Vergleichen von fluoreszierenden Signalen der Detektionslinie und der Kontrolllinie, um das Schädel-Hirn-Trauma zu diagnostizieren.

8. Das Verfahren nach Anspruch 7, umfassend:
Diagnostizieren des Schädel-Hirn-Traumas in Kombination mit einer Glasgow-Koma-Skala (GCS) (Wachsein, Sprachfunktion und Bewegungsfunktion).

## Revendications

1. Un dispositif d'analyse de flux latéral par fluorescence à résolution temporelle (10) comprenant :
dans un dispositif d'analyse de flux latéral capable de détecter un marqueur de lésion cérébrale traumatique,
un tampon d'échantillon (11) dans lequel un échantillon de sang contenant un marqueur de lésion cérébrale traumatique est injecté,
un tampon d'adsorption (13) incluant une sonde qui est mélangée au marqueur lorsque le marqueur de lésion cérébrale traumatique migre du tampon d'échantillon pour former un complexe de marqueur de lésion cérébrale traumatique, et
un film poreux (18) qui est en communication fluidique avec le tampon d'adsorption et fait migrer par capillarité le complexe de marqueur de lésion cérébrale traumatique du tampon d'adsorption vers une ligne de détection (20),
dans lequel la sonde inclut un anticorps de capture (7) consistant en un anticorps (3) marqué avec un matériau de liaison spécifique se liant spécifiquement au marqueur de lésion cérébrale traumatique et un anticorps détecteur (8) consistant en un anticorps marqué avec un matériau fluorescent (6) présentant une durée de vie d'émission relativement longue de 1 microseconde ou plus,
dans lequel le marqueur de lésion cérébrale traumatique est une protéine acide fibrillaire gliale (GFAP), et l'un quelconque parmi S100B, UCH-L1, NSE, NeuN, CNPase, CAM-1, iNOS, MAP-1, MAP-2, SBDP145, SBDP120, la III-tubuline, une protéine synaptique, la neuroserpine, l'internexine, LC3, la neurofacine, EAAT, DAT, la nestine, la cortine-1, CRMP, ICAM-1, ICAM-2, ICAM-5, VCAM-1, NCAM-1, NCAM-L1, NCAM-120, NCAM-140, NL-CAM, AL-CAM ou C-CAM1,
dans lequel la biotine est utilisée comme le matériau de liaison spécifique, l'europium est utilisé comme le matériau fluorescent, et un mélange d'un anticorps d'origine murine et d'un anticorps d'origine lapin est utilisé comme anticorps pour l'anticorps détecteur.

2. Le dispositif d'analyse de flux latéral pour une lésion cérébrale traumatique au moyen d'une analyse de fluorescence à résolution temporelle selon la revendication 1, dans lequel
le tampon d'adsorption inclut un premier (14) et un second (15) tampon d'adsorption fournis séquentiellement par la sonde, dans lequel le premier tampon d'adsorption fournit un matériau de liaison spécifique (2) formant l'anticorps de capture et le second tampon d'adsorption fournit le matériau fluorescent formant l'anticorps détecteur.

3. Le dispositif d'analyse de flux latéral pour une lésion cérébrale traumatique au moyen d'une analyse de fluorescence à résolution temporelle selon la revendication 1, dans lequel
un mélange d'au moins deux anticorps provenant d'espèces différentes est utilisé comme anticorps marqué avec le matériau de liaison spécifique ou le matériau fluorescent.

4. Le dispositif d'analyse de flux latéral pour une lésion cérébrale traumatique au moyen d'une analyse de fluorescence à résolution temporelle selon la revendication 1, dans lequel
dans un anticorps lié à des particules d'europium, la teneur en anticorps d'origine lapin est à une concentration de 0,1 % à moins de 2 %, de préférence 1 % dans une solution de pulvérisation sur tampon d'adsorption.

5. Le dispositif d'analyse de flux latéral pour une lésion cérébrale traumatique au moyen d'une analyse de fluorescence à résolution temporelle selon la revendication 4, dans lequel
dans l'anticorps lié à des particules d'europium, l'anticorps d'origine murine est en outre ajouté à une concentration de 3 % à 12 %, de préférence 3 % dans la solution de pulvérisation sur tampon d'adsorption.

6. Le dispositif d'analyse de flux latéral pour une lésion cérébrale traumatique au moyen d'une analyse de fluorescence à résolution temporelle selon la revendication 5, dans lequel
la GFAP, qui est l'un des marqueurs de lésion cérébrale traumatique, est détectée avec une sensibilité inférieure à 100 pg/mL en utilisant l'anticorps d'origine lapin comme anticorps de capture.

7. Un procédé de diagnostic d'une lésion cérébrale traumatique utilisant un dispositif d'analyse de flux latéral (10) au moyen d'une analyse de fluorescence à résolution temporelle selon l'une quelconque des revendications 1 à 6, le procédé comprenant les étapes de :
préparer (S10) un échantillon de sang contenant un marqueur de lésion cérébrale traumatique,
injecter (S20) l'échantillon de sang contenant le marqueur de lésion cérébrale traumatique dans un tampon d'échantillon (11),
former (S30) un complexe de marqueur de lésion cérébrale traumatique (20a) consistant en un anticorps de capture (7) marqué avec un matériau de liaison spécifique (2) et d'un anticorps détecteur (8) marqué avec un matériau fluorescent (6) tout en faisant migrer l'échantillon de sang contenant le marqueur de lésion cérébrale traumatique le long d'un tampon d'adsorption (13) adjacent au tampon d'échantillon par un phénomène capillaire,
faire migrer (S40) le complexe de marqueur de lésion cérébrale traumatique le long d'un film poreux (18) en communication fluidique avec le tampon d'adsorption pour le lier à un matériau de capture (5) sur une ligne de détection (20) du film poreux,
lier (S50) toute sonde (22b) ne se liant pas au marqueur de lésion cérébrale traumatique à un matériau de capture d'une ligne de commande (22) à travers la ligne de détection, et
mesurer (S60) une concentration du marqueur de lésion cérébrale traumatique en irradiant de la lumière sur la ligne de détection et la ligne de commande à partir d'un testeur de fluorescence à résolution temporelle (50) et comparer les signaux fluorescents de la ligne de détection et de la ligne de commande pour diagnostiquer la lésion cérébrale traumatique.

8. Le procédé selon la revendication 7, comprenant :
le diagnostic de la lésion cérébrale traumatique en combinaison avec une échelle de Glasgow (GCS) (éveil, fonction verbale et fonction motrice).
